# EUROPEAN PATENT SPECIFICATION

(11) **EP 0 788 809 B1**
(45) Date of publication and mention of the grant of the patent: **03.05.2006**
(21) Application number: 96830623.3
(22) Date of filing: 13.12.1996
(51) Int. Cl.: A61N 1/20, A61N 1/32

(54) **A portable device for treating insect bites and the like**
Tragbare Vorrichtung zur Behandlung von Insektenstichen oder dergleichen
Appareil portatif pour traiter les piqûres d'insectes et autres

(30) Priority: 19.01.1996 IT MI960085
(43) Date of publication of application: 13.08.1997
(73) Proprietor: TECNIMED S.r.l., 21040 Vedano Olona (Varese) (IT)
(72) Inventor: Bellifemine, Francesco, 21100 Varese (IT)
(74) Representative: Righetti, Giuseppe

(56) References cited:
- WO-A-87/04068
- US-A- 4 297 609
- US-A- 4 741 347
- US-A- 4 873 609
- US-A- 4 982 743
- US-A- 5 496 356

## Description

The present invention relates to a portable device for treating insect bites and the like.

It is known that at the present time there is a wide spread of many products and devices which are adapted for treatment of mosquito, wasp and bee bites or even more severe bites such as those produced by scorpions and the like, vipers or other animals.

In particular, there are many products based on ammonia, commercialized either in the form of a cream or in the form of a stick, which however are scarsely able to soothe irritation caused by bites and, in addition, they have effects limited in time.

Beside the products briefly described above, spreading of devices has also occurred which are capable of treating the bitten area by heating until 50-60°C (in this connection see document FR-A-1139096), as well as of devices capable of producing passage of a low-voltage direct current directly from a small storage battery to the bitten area (see document US-4982743-PEARSON).

The above devices however are of little flexibility because a rather long period of time is required for reaching a good result in terms of bite treatment.

Obviously due to the above limitation, it is also impossible for several persons to take advantage of the devices in question simultaneously.

It is also to point out that, in any case, the concerned devices need to be provided with a storage battery the cost of which is not negligible and in addition periodical recharging of same is to be carried out by a user in order to ensure a correct operation of the device itself.

First of all, the required circuit for carrying out raising of the electric voltage has such an intrinsic cost that the overall cost of the devices becomes greatly increased.

In addition, due to the intensity of the electric discharge thus produced, use of these devices is only justified when very serious cases are to be treated, such as insect bites produced by wasps, bumble-bees or scorpions or in case of snake bites.

At all events, these devices are adapted for use on adults only because the electric discharge has such an intensity that it is rather painful by itself and therefore can be hardly used on children.

In addition, it will be recognized that for these devices too the use of storage batteries is required, which will bring about an increase in prices and, as already said above, the necessity to periodically recharge or replace said storage batteries in order to enable a correct operation of the device.

It is also known from documents WO 8704068 and US 4741347 an electric pulses generating apparatus for therapeutic applications, in which a piezoelectric generator is connected to electrodes. Some of the electrodes at least are placed in contact or in vicinity of a skin of a patient to be treated. A current of piezoelectric origin in caused to flow through a distribution head formed with notches. The piezoelectric element is activated by a push button movable transversal to the longitudinal extension of the apparatus.

Moreover, it is also known from document US 4297609 a high-voltage generating device generating a high-voltage in response to impartation of an impact to a piezoelectric element. The piezoelectric element and the push button which activates said piezoelectric element are disposed along the longitudinal extension of the device.

Under this situation, the present invention aims at providing a new portable device for treating insect bites and the like, capable of overcoming the above mentioned drawbacks.

In particular, it is a fundamental object of the invention to provide a device capable of operating in a correct manner without requiring periodical rechargings.

Another object of the invention is to provide a device of easy and ready setting so that its use may be very pratical and intuitive.

A further object of the invention is to provide a device of simple structure and easy and cheap manufacture and above all a device for the construction of which pieces that can be easily found on the market are to be used.

Another important object of the invention is to provided a device capable of producing electric discharges appropriate for small-insect bites and therefore adapted for repeated use on childred too without involving any risks and without causing too much worry or pain.

The foregoing and further objects that will become more apparent in the progress of the present description are substantially achieved by a device for treating insect bites and the like, as disclosed in the appended claims.

Further features and advantages will best be understood from the detailed description of some preferred embodiments of a portable device in accordance with the present invention, taken hereinafter by way of nonlimiting example with reference to the accompanying drawings, in which:
- Fig. 1 is a partial perspective view of a device for treating insect bites ;
- Fig. 2 is a longitudinal view, partly in section, of the representation taken in Fig. 1;
- Fig. 3 is a partly sectional longitudinal view of a second embodiment of such a device taken as a whole;
- Fig. 4 is a front view of the device shown in Fig. 3;
- Fig. 5 is a partly sectional top view of the device shown in Fig. 3;
- Fig. 6 is a partly sectional longitudinal view of a third device;
- Figs. 7a and 7b are two views at right angles to each other and partly in section of a fourth device;
- Fig. 8 is a partial front view of a detail 1 to be associated with the device in question;
- Fig. 9 is a sectional view taken along line IX-IX in Fig. 8;
- Fig. 10 is a view showing an alternative form of a detail concerning the device shown in Figs. 1 to 5;
- Figs. 11 to 13 are perspective views of the device in accordance with the invention;
- Fig. 14 is a view showing one of the shells of the device in Fig. 13;
- Fig. 15 is a view showing one of the two shells forming the device in Fig. 12; and
- Fig. 16 is a cross-sectional view taken along line XVI-XVI in Fig. 11.

With reference to the drawings, a portable device for treating insect bites or bites produced by other animals has been generally identified by reference numeral 100.

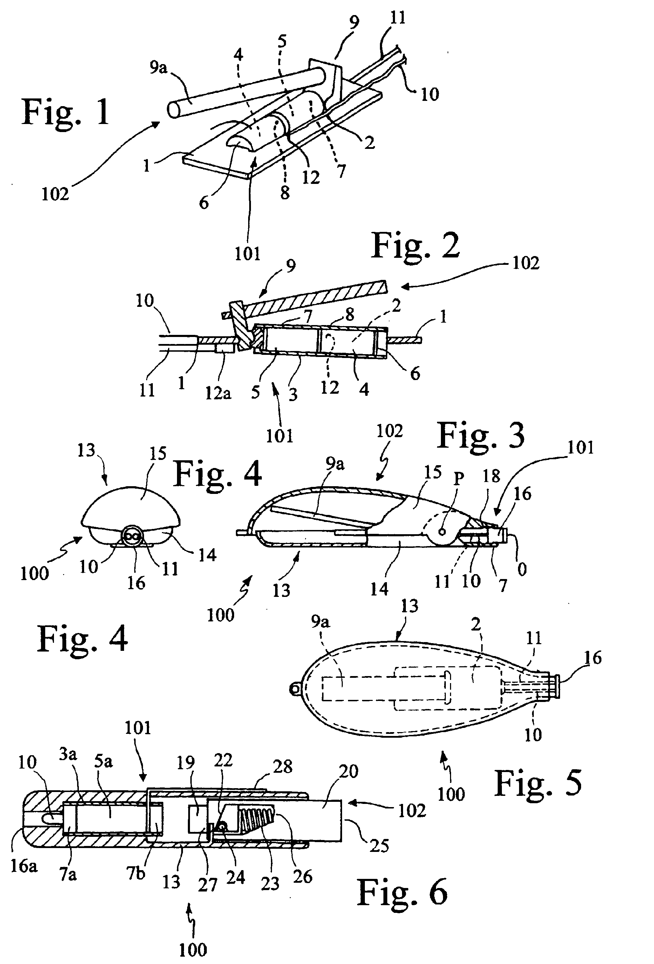

In more detail and referring particularly to the accompanying drawings, it is to note that Figs. 1 and 2 show the inner components of one embodiment of the device in question.

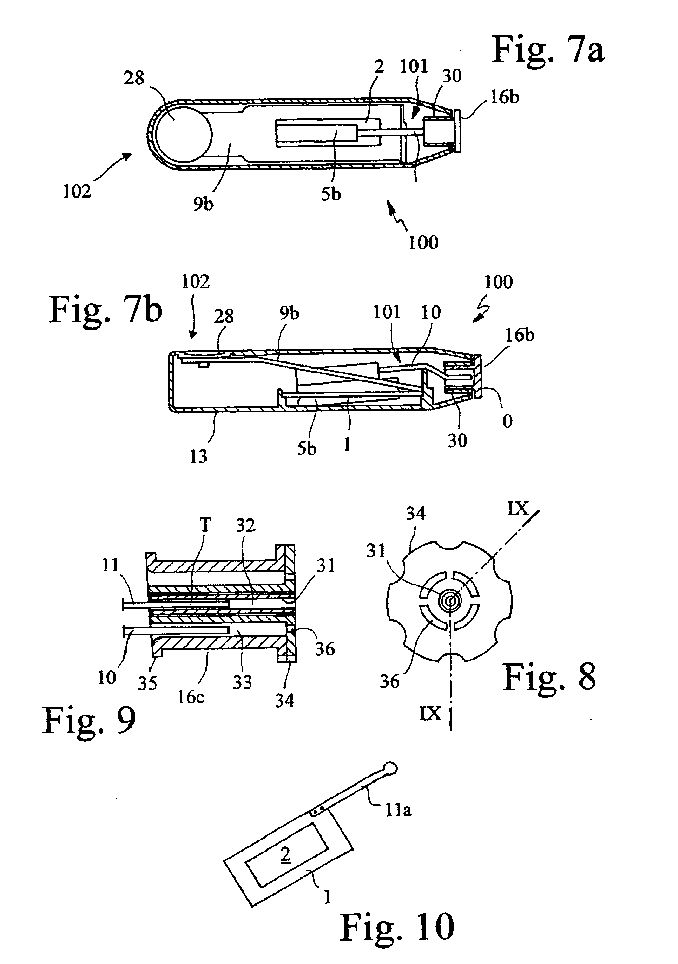

As viewed from said figures, such inner components comprise voltage-generating means 101 defined by an armature 1 preferably in the form of a steel plate, which has a rectangular cavity 2 in the middle (see Figs. 5 and 7a) inside which a cylindrical case 3 made of plastic material is housed; this case has two circular opposite ends that are open and hold two piezoelectric bodies in the form of ceramic cylinders 4 and 5. Each of said cylinders 4 and 5 is covered at its outer end with a steel disc 6, 7 and is separated from the other cylinder by a brass disc 8. The portable device comprises drive means 102 which is herein defined by a manual contrivance consisting of a lever system 9 which, once operated, acts on the steel disc 7 to press the two piezoelectric bodies. In this way, a voltage is induced at the ends of the piezoelectric bodies so that they deliver several electric discharges that are picked up, through the brass disc 8, by a first conductor means 10 having one end welded at 12 to said brass disc. A second conductor means 11 has one end welded at 12a to the armature 1. The first and second conductor means 10 and 11 therefore are true wire-like conductors and have active ends (electrodes) adapted to operate close to or directly in contact with the user's area to be treated.

As shown in Figs. 3, 4 and 5, the inner components briefly described above are housed in a casing 13 consisting of two shells 14 and 15 articulated on each other at a pivot pin P close to a mouth 16 at which the free ends of conductors 10 and 11 converge. When electric discharges are to be produced, grasping of the casing 13 is carried out by a user who presses the two shells 14, 15 against each other; said shells by angularly moving close to each other cause operation of lever 9a.

As shown in Fig. 3, the mouth 16 which is entirely held within one of said two shells, can take two positions at the casing 13 end. An annular extension 17 of mouth 16 can be fitted with force into a first or a second annular groove 18 formed in the two shells, while the position of conductors 10 and 11 remains unchanged. In this way the distance of the two conductor ends from the outer rim 10 of mouth 16 can be of two or three millimetres. As an alternative solution, the mouth 16 may be provided to be mounted on casing 13 by a continuous adjustment system substantially enabling the mouth to take an indefinite number of relative positions with respect to the casing so that operation of the device is conformed to the different requirements of use.

Alternatively, Fig. 1 shows a steel armature 1 carrying the cavity 2 and an extension 11a in the form of an elastic blade with a spherical end acting as a second conductor.

Shown in Fig. 6 is another embodiment of such a device in which the drive means 102 is defined by a striking device causing an electric discharge from a ceramic cylinder 5a (piezoelectric body defining the voltage-generating means) held within a plastic case 3a covered at its ends with two steel discs 7a, 7b. A striking hammer 19 is partially housed in a hollow cylindrical cap 20 retained at the end of casing 13 in the device which comprises an opening having two inclined walls 22 and 23 converging in the vicinity of a pin 24 passing through the striking hammer 19. The outer end 25 of the cylindrical cap acts as a push-button. In addition, interposed between the cap end 25 and the striking hammer 19 is a spring 26 which can be in a compressed condition when the push-button is pressed and which pushes the striking hammer. Immediately after beginning of a compression, the pin 24 bears against a shoulder 27 integral with casing 13. Subsequently, as compression goes on, the pin 24 slides along the inclined wall 23 reaching and going beyond the upper end of shoulder 27. At this point the striking hammer is pushed with violence against the steel disc 7b and produces delivery of an electric discharge. The figure also shows conductor 10 with its end positioned, by way of example, at 2.5 mm from the mouth rim 16a and a strap 28 laid down on casing 13 and connected to the ceramic cylinder 5a to work as a ground strap.

For the sake of simplicity, the figure does not show a spring the task of which is to bring the striking hammer back to the starting position.

Figs. 7a and 7b show another device in which the lever system comprises a lever 9b and a push-button 28 fastened to one end of said lever. Both the lever 9b and push-button 28 are made of a conductive material and define said drive means and also, in this case, the second conductor means.

The lever can press the piezoelectric cylinder 5b held within a case not shown, in turn located inside the cavity 2 of armature 1. In this way as a person applies pressure to push-button 28 with a finger, an electric discharge is produced at the end of conductor 10. The electric discharge practically closes the electric circuit defined between the finger, the user's body, the application point of the device and the first conductor 10 (this is obtained by virtue of the conductive properties of lever 9b and push-button 28).

Also shown is a mouth 16b which is to be caused to slide by friction within a cylindrical housing 30 so as to vary the distance of the conductor 10 end from the outer mouth rim 0.

Figs. 8 and 9 also show a mouth 16c conveniently arranged so as to adjust the distance of one conductor alone, for example conductor 10, whereas the electric contact of conductor 11 with the skin takes place always directly. Actually, even if position of mouth 16c within the device end is varied, a copper tube 31 housed within the central hole 32 of said mouth and extending until the outer rim of the latter is always in contact with conductor 11 which is longitudinally fixed, bare along its end portion T and loose so as to always touch the copper tube 31.

Located around the central hole 32 is a chamber 33 inside which conductor 10 is housed under any angular position of the hand-wheel 34. Position of mouth 16c within the casing end (not shown) is defined by making it rotate by means of the handwheel 34 in either direction, so that the helical tooth may slide in a corresponding helical groove (not shown) formed at one end of the device casing.

Openings 36 on the front wall of the mouth 16c enable the electric circuit between the first conductor 10, the user's skin and the second conductor 11 to be closed.

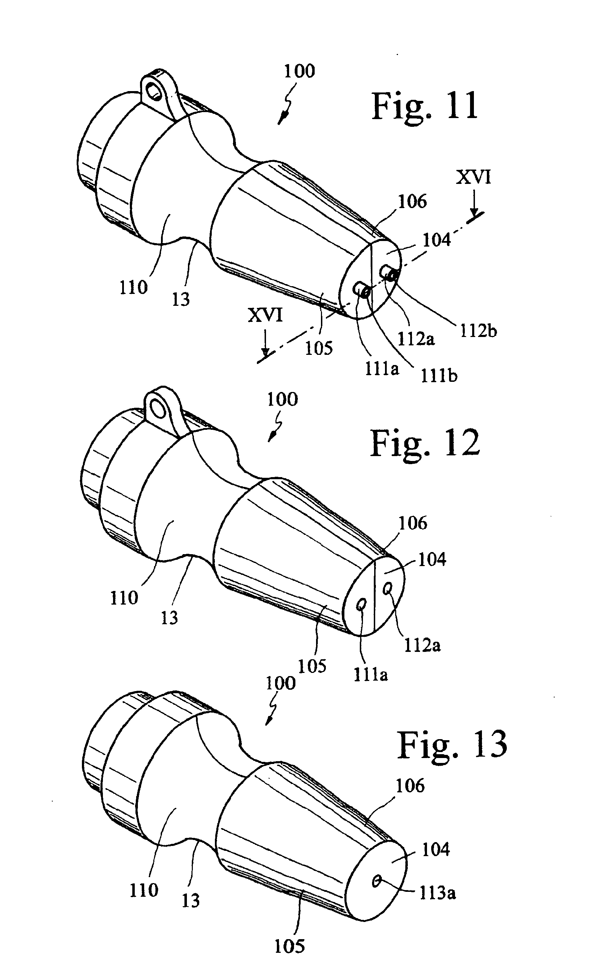

Finally, Figs. 11 to 15 show the preferential solution in accordance with the invention in which the casing 13 is provided to be defined by two shells 105 and 106 to be mutually engaged and preferably quite identical with each other so that a single mould is required for their manufacture. As viewed from said figures, the drive means is defined by a spring striker 107 (of the same type as described with reference to the embodiment shown in Fig. 6, for example) acting on the voltage-generating means 101 consisting of a piezoelectric body 108 disposed inside the casing.

As can be seen, the mutually-engaged shells define a sliding seat 109a for the striker which is provided with a driving push-button 109 emerging at least partly from the casing so that it can be activated by a user.

Advantageously, the conformation of each shell also defines an annular engagement seating 110 disposed at a longitudinally intermediate area of the shells to enable the device to be easily handled like a syringe.
In this embodiment, the first and second conductor means can be both defined by true conductive wires 111, 112 the ends 111a, 112a of which partly emerge from, or are disposed substantially flush with an operating end face 104 of said casing (see in this connection Figs. 11 and 12). It is to note that, in this case, terminal elements 111b, 112b of substantially cylindrical conformation (see Fig. 11) can be preferably associated with the conductive wire ends.

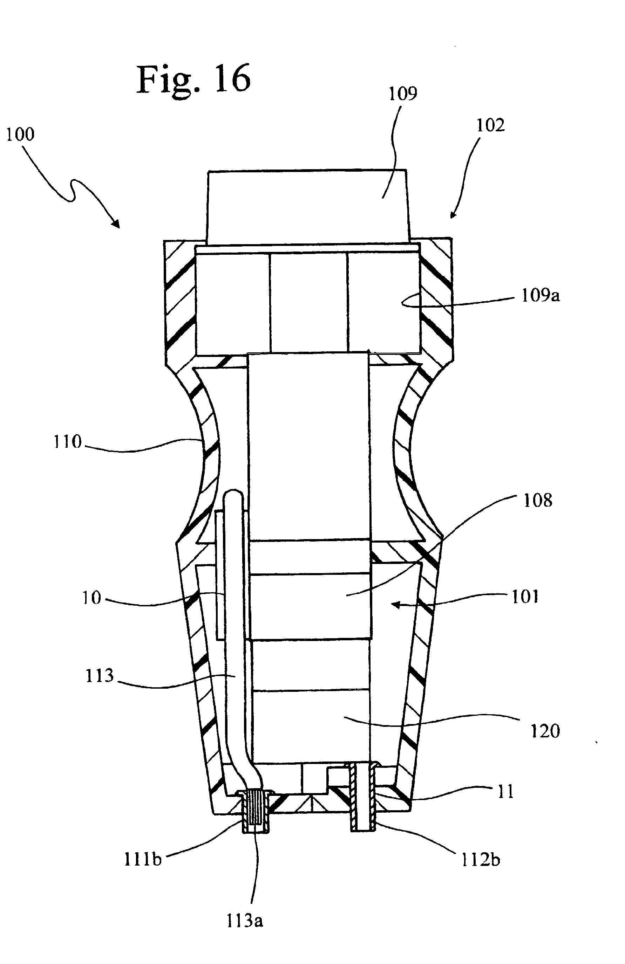

It should be pointed out that, as on the other hand clearly apparent from Fig. 16, one of the conductor means 10, 11 can be wholly defined by one of said terminal elements 111b, 112b. In more detail a conductive wire will 1 be in this case connected at one end with the piezoelectric body while, at the other end 113a, it will be provided with a terminal element; the other terminal element 112b of an axial extension greater than the former terminal element, will be directly connected with a conductive body 120 also electrically connected to the piezoelectric body.

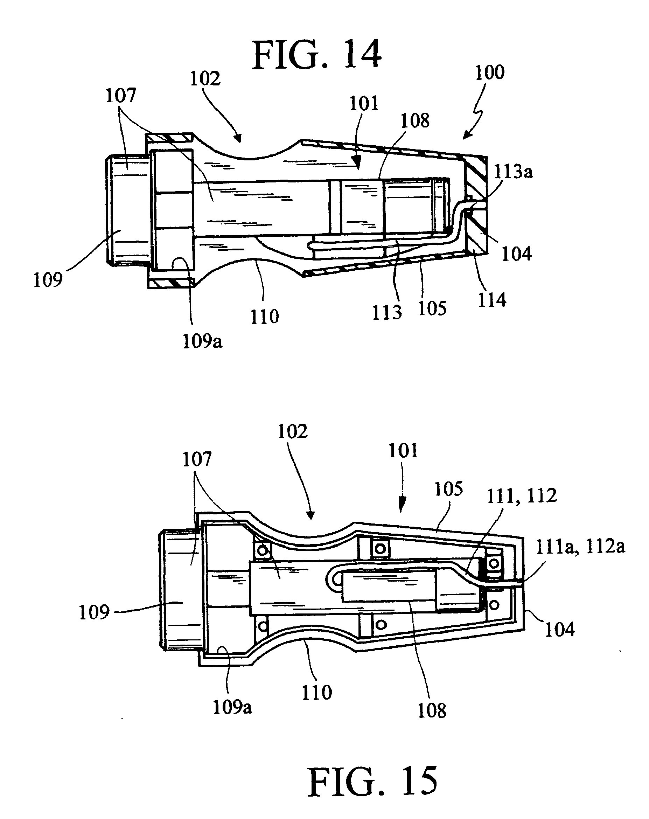

Alternatively, as shown in Figs. 13 and 14, it is provided that the first conductor means alone be defined by a true conductive wire or wire-like conductor 113 having one end 113a disposed flush with (see Fig. 13) or partially emerging from, or located backwards relative to the end face of the casing (not shown), whereas the second conductor means is defined by the push-button which is made of an electrically conductive material and electrically connected to the piezoelectric body.

In this case the circuit is defined by the first conductor means, the piezoelectric body, the striker, the push-button, the user's body (thumb, hand, arm, trunk, area hit by a bite) and closes on the end 113a of the first conductor means.
Therefore the electric discharge in this case is directed transversely of the area submitted to treatment and it efficiently passes through the poison or other serum injected by the insect.

Preferably, the second conductor means comprises casing 13 too which can be made of electrically conductive material and is brought into electric connection with the striker or directly with the piezoelectric body on the opposite side from where the first conductor means is connected. In this case, for insulation reasons, in order to avoid an electric discharge directly between the end 113a of the first conductor means and the casing, a coating portion 114 of insulating material is advantageously associated with said casing 13 and is disposed at one casing end opposed to the striker or at said end face.

The invention achieves important advantages.

Firstly, by adopting a very simple and therefore inexpensive structure and with the use of the piezoelectric body, a device has been made available which is capable of treating insect bites and bites produced by other similar animals which can be used repeatedly, has a substantially infinite self-containedness and causes electric discharges of few kW and more precisely until a maximum value of about 13 kV (from a technical point of view discharges of higher voltage could be achieved, but they would be felt to a greater extent by the user).

By virtue of its simple structure, the device in question can be carried by a user without occupying too much room, is very light-in-weight and very practical in use, above all 1 when it is made in the form of a striker, as shown in Figs. 11-15.

In addition, particularly advantageous are those configurations in which the first conductor means alone is defined by a conductive wire and has one end directly active on the user's skin, whereas the second conductor means is defined, depending on each individual case, by the casing, the striker, the push-button, so that the end of the first conductor means is ground-closed on the user's body thereby causing a transverse discharge onto the area to be treated.

These embodiments are particularly advantageous because, due to the relatively low intensity of the concerned voltages (about 13 kv), it is preferable for the electric discharge to pass transversely substantially through the whole area hit by a bite.

The invention is also advantageous in its most specific aspects.

Actually, by the use of a conductive-material casing, in the handgrip area the charge intensity per unit surface passing through the user's portion directly in contact with the device is greatly reduced. In addition, in this case, the use of the coating portion of insulating material is very advantageous: actually, under use conditions, it ensures the substantial impossibility of an electric discharge occurring between the casing and the end of the first conductive wire. On the contrary, it is to note that when the device is operated in vain, due to the fact that the sizes of the coating portion can be established in a precise manner, closure of the circuit on the casing is obtained, which will bring about protection of the piezoelectric body that otherwise would undergo a dangerous discharge at its inside.

It is also important to note that a certain importance can be attached to the use of the mouth associated at its end with the casing which enables the distance of one or both ends of the conductive wires to be varied relative to the body surface to be treated. This, in some cases, has proved to be advantageous for adjusting the discharge intensity so as to adapt it both to the bite nature and the user's particular sensitivity.

Obviously many modifications and variations can be made to the invention as conceived, all of them falling within the scope of the appended claims.

## Claims

1. A portable device for treating insect bites and the like comprising:
- voltage-generating means (101) comprising at least one piezoelectric body (108);
- drive means (102) arranged for activation of said voltage-generating means comprising at least one manual device capable of applying a predetermined force to the piezoelectric body to cause arising of a corresponding voltage at the ends of the piezoelectric body;
- a casing (13) housing said piezoelectric body (108) and presenting an operating end face (104) the manual device comprising a driving push button (109) emerging at least partly from the casing so that it can be activated by a user;
- a first and a second conductor means (10,11) electrically connected to at least one piezoelectric body to be charged with said voltage and, under operating conditions, to close the circuit with said area of the user's body submitted to treatment; and
- an end (111a; 113a) of the first conductor means (10) being disposed at the operating end face (104) of the casing (13) ; and
an end (112b) of the second conductor means (11) being disposed at the operating end face, or the second conductor means being defined by the push-button (109) or by the casing (13);
**characterized in that** the driving push button (109), the piezoelectric body (108) and the end (111a; 113a) of the first conductor means (10) are consecutively disposed along a longitudinal extension of the portable device.

2. A portable device as claimed in claim 1, **characterised in that** the manual device is comprised of at least one striker (19; 107) arranged to be released under the thrust of a spring element (26) loaded by the user to hit said piezoelectric body and exert said predetermined force necessary to produce said voltage.

3. A portable device as claimed in claim 1, **characterized in that** the first conductor means (10) comprises a conductive wire and **in that** the second conductor means (11) comprises at least the drive means (102) which is made of an electrically conductive material.

4. A portable device as claimed in claim 1, **characterised in that** the holding casing (13) is made of an electrically conductive material.

5. A portable device as claimed in claim 1, **characterized in that** it comprises a coating portion (114) of insulating material operatively associated with one end of said casing opposed to the striker.

6. A portable device as claimed in claim 1, **characterised in that** said second conductor means (11) is defined by a conductive wire.

7. A portable device as claimed in claim 1, **characterized in that** said second conductor means (11) comprises a terminal element (112) brought into electrical connection with the piezoelectric body (108).

8. A portable device as claimed in claim 7, **characterized in that** the first conductor means (10) comprises a conductive wire (113) provided at one end with a terminal element (111b) having an axial extension smaller than the terminal element (112b) defining said second conductor means.

## Revendications

1. Appareil portatif pour traiter les piqûres d'insectes et autres, comprenant:
- des moyens générateurs de tension (101) comprenant au moins un corps piézo-électrique (108);
- des moyens d'entraînement (102) destinés à l'activation desdits moyens générateurs de tension comprenant au moins un dispositif manuel en mesure d'appliquer une force prédéterminée au corps piézo-électrique pour causer la naissance d'une tension correspondante, aux extrémités du corps piézo-électrique;
- un boîtier (13) logeant ledit corps piézo-électrique (108) et présentant une face opérante extrême (104), le dispositif manuel comprenant un poussoir de commande (109) faisant saillie au moins partiellement du boîtier de sorte qu'il peut être activé par un utilisateur;
- des premiers et deuxièmes moyens conducteurs (10, 11) connectés électriquement au moins à un corps piézo-électrique qui doit être chargé par ladite tension et, en conditions opérantes, pour fermer le circuit avec ladite aire du corps de l'utilisateur soumise au traitement; et
- une extrémité (111a; 113a) des premiers moyens conducteurs (10) étant disposée en correspondance avec la face extrême opérante (104) du boîtier (13); et
- une extrémité (112b) des deuxièmes moyens conducteurs (11) étant disposée en correspondance avec la face extrême opérante, ou les deuxièmes moyens conducteurs étant définis par le poussoir (109) ou par le boîtier (13) ;
**caractérisé en ce que** le poussoir de commande (109), le corps piézo-électrique (108) et l'extrémité (111a, 113a) des premiers moyens conducteurs (10) sont disposés consécutivement le long d'une extension longitudinale de l'appareil portatif.

2. Appareil portatif selon la revendication 1, **caractérisé en ce que** le dispositif manuel comporte au moins une masse (19; 107) destinée à se déclencher sous la poussée d'un élément à ressort (26) armé par l'utilisateur pour frapper ledit corps piézo-électrique et exercer ladite force prédéterminée, nécessaire pour produire ladite tension.

3. Appareil portatif selon la revendication 1, **caractérisé en ce que** les premiers moyens conducteurs (10) comportent un fil conducteur et **en ce que** les deuxièmes moyens conducteurs (11) comportent au moins les moyens d'entraînement (102) qui sont réalisés en une matière électriquement conductrice.

4. Appareil portatif selon la revendication 1, **caractérisé en ce que** le boîtier de logement (13) est fabriqué en une matière électriquement conductrice.

5. Appareil portatif selon la revendication 1, **caractérisé en ce qu'**il comporte une portion de revêtement (114) de matière isolante associée de manière opérationnelle à une extrémité dudit boîtier opposée à la masse.

6. Appareil portatif selon la revendication 1, **caractérisé en ce que** lesdits deuxièmes moyens conducteurs (11) sont définis par un fil conducteur.

7. Appareil portatif selon la revendication 1, **caractérisé en ce que** lesdits deuxièmes moyens conducteurs (11) comportent un élément terminal (112) qui est connecté électriquement au corps piézo-électrique (108).

8. Appareil portatif selon la revendication 7, **caractérisé en ce que** les premiers moyens conducteurs (10) comportent un fil conducteur (113) muni à une extrémité d'un élément terminal (111b) ayant une extension axiale plus petite que l'élément terminal (112b) définissant lesdits deuxièmes moyens conducteurs.

## Patentansprüche

1. Tragbare Vorrichtung zur Behandlung von Insektenstichen oder dergleichen, umfassend:
- Spannungserzeugermittel (101), umfassend mindestens einen piezoelektrischen Körper;
- Antriebsmittel (102), die zur Aktivierung der Spannungserzeugermittel bereitgestellt sind und mindestens eine manuelle Einrichtung umfassen, die in der Lage sind, am piezoelektrischen Körper eine vorgegebene Kraft anzuwenden, um an den Enden des piezoelektrischen Körpers das Aufkommen einer entsprechenden Spannung zu bewirken;
- ein Gehäuse (13), das den piezoelektrischen Körper (108) aufnimmt und eine Endarbeitsfläche (104) aufweist, wobei die manuelle Einrichtung eine Betätigungsdrucktaste (109) umfasst, die mindestens teilweise derart aus dem Gehäuse vorsteht, dass sie durch einen Benutzer aktiviert werden kann;
- erste und zweite Leiter (10, 11), die mindestens mit einem mit der Spannung zu ladenden piezoelektrischen Körper elektrisch verbunden sind und bei Arbeitsbedingungen, um den Stromkreis der genannten der Behandlung des Körpers des Benutzers unterzogene Fläche zu schließen; und
- wobei ein Ende (111a; 113a) der ersten Leitermittel (10) im Bereich der Arbeitsendfläche (104) des Gehäuses (13) angeordnet ist; und
- wobei ein Ende (112b) der zweiten Leitermittel (11) im Bereich der Arbeitsendfläche angeordnet ist oder die zweiten Leitermittel durch die Drucktaste (109) oder durch das Gehäuse (13) festgelegt sind;
**dadurch gekennzeichnet, dass** die Betätigungsdrucktaste (109), der piezoelektrische Körper (108) und das Ende (111a, 113a) der ersten Leitermittel (10) nacheinander längs einer Längsausdehnung der tragbaren Vorrichtung angeordnet sind.

2. Tragbare Vorrichtung nach Anspruch 1, **dadurch gekennzeichnet, dass** die manuelle Einrichtung einen ersten Schlaghammer (19; 107) umfasst, der bereitgestellt ist, um unter dem Schub eines durch den Benutzer gespannten Federelementes (26) zu schnappen, um den piezoelektrischen Körper zu treffen und die vorgegebene Kraft auszuüben, die zur Erzeugung der Spannung notwendig ist.

3. Tragbare Vorrichtung nach Anspruch 1, **dadurch gekennzeichnet, dass** die ersten Leitermittel (10) einen Leiterdraht umfassen und **dadurch**, dass die zweiten Leitermittel (11) mindestens die Betätigungsmittel (102) umfassen, die in einem elektrisch leitenden Material ausgeführt sind.

4. Tragbare Vorrichtung nach Anspruch 1, **dadurch gekennzeichnet, dass** das Gehäuse (13) aus einem elektrisch leitfähigen Material besteht.

5. Tragbare Vorrichtung nach Anspruch 1, **dadurch gekennzeichnet, dass** sie einen Mantelabschnitt (114) aus einem Isoliermaterial umfasst, der wirksam einem Ende des Gehäuses zugeordnet ist, das dem Schlaghammer abgewandt ist.

6. Tragbare Vorrichtung nach Anspruch 1, **dadurch gekennzeichnet, dass** die zweiten Leitermittel (11) durch einen Leiterdraht festgelegt sind.

7. Tragbare Vorrichtung nach Anspruch 1, **dadurch gekennzeichnet, dass** die zweiten Leitermittel (11) ein Endelement (112) umfassen, das mit dem piezoelektrischen Körper (108) elektrisch verbunden ist.

8. Tragbare Vorrichtung nach Anspruch 7, **dadurch gekennzeichnet, dass** die ersten Leitermittel (10) einen Leiterdraht (113) umfassen, der an einem Ende mit einem Endelement (111b) versehen ist, das eine axiale Ausdehnung besitzt, die kleiner ist als das Endelement (112b), das die zweiten Leitermittel festlegt.
